# EUROPÄISCHE PATENTANMELDUNG

(11) **EP 2 810 640 A1**
(43) Veröffentlichungstag der Anmeldung: **10.12.2014**
(21) Anmeldenummer: 13170256.5
(22) Anmeldetag: 03.06.2013
(51) Int. Cl.: A61K 8/365, A61Q 19/10, C07C 69/675, C07C 69/68, C07C 69/70, C07C 59/01, C07C 59/06, C07C 59/08

(54) **Ester von Oligohydroxycarbonsäuren und deren Verwendung**

(71) Anmelder: BASF SE, 67056 Ludwigshafen (DE)
(72) Erfinder: Behler, Ansgar, 46240 Bottrop (DE)
(74) Vertreter: Reitstötter Kinzebach

(57) **Zusammenfassung**

Die vorliegende Erfindung betrifft Ester von Oligohydroxycarbonsäuren, kosmetische und pharmazeutische Mittel, die diese Ester enthalten sowie die Verwendung dieser Ester als Verdickungsmittel, speziell für tensidhaltige Zusammensetzungen.

## Beschreibung

### HINTERGRUND DER ERFINDUNG

Die vorliegende Erfindung betrifft Ester von Oligohydroxycarbonsäuren, kosmetische und pharmazeutische Mittel, die diese Ester enthalten sowie die Verwendung dieser Ester als Verdickungsmittel, speziell für tensidhaltige Zusammensetzungen.

### STAND DER TECHNIK

Verdickungsmittel dienen dazu, die Viskosität fließfähiger Zusammensetzungen zu erhöhen. Verdickungsmittel werden in großem Umfang zur Modifizierung der rheologischen Eigenschaften von wässrigen Zubereitungen eingesetzt, beispielsweise auf den Gebieten der Kosmetik, Pharmazie, Wasch- und Reinigungsmittel, Human- und Tiernahrung, etc. Übliche Verdickungsmittel, wie sie vor allem in der Kosmetik eingesetzt werden, sind beispielsweise Fettalkoholalkoxilate, wie die Ethoxilate des Laurylalkohols (INCI-Bezeichnung Laureth x, wobei x für den Alkoxilierungsgrad steht) oder die Ester von Polyolalkoxilaten mit Fettsäuren, wie z. B. die Trioleate von ethoxiliertem und/oder propoxiliertem Trimethylolpropan.

Für viele aktuelle Anwendungen werden an Verdickungsmittel außer einer guten verdickenden Wirkung weitere Anforderungen gestellt. Insbesondere in der Kosmetik ist es häufig erwünscht, dass die eingesetzten Wirk- und Hilfsstoffe gleichzeitig mehrere Aufgaben erfüllen, beispielsweise zusätzlich zur verdickenden Eigenschaft auch schaumstabilisierende Eigenschaften aufweisen oder als Solubilisatoren für andere Komponenten, wie UV-Filter, dienen. Des Weiteren besteht ein Bedarf an Verdickungsmitteln, die zumindest teilweise aus biogenen Quellen und speziell auch nachwachsenden Rohstoffen hergestellt werden können. Weiterhin besteht auch ein Bedarf an Verdickungsmitteln, die keine alkoxilierten Gruppen aufweisen und die somit insbesondere den Einsatz von Ethylenoxid zu ihrer Herstellung überflüssig machen.

Die DE 40 03 096 A1 beschreibt sulfatierte Hydroxycarbonsäureester und deren Verwendung als oberflächenaktive Substanzen. Das auf Seite 4 dieses Dokuments beschriebene Beispiel A zur Herstellung von Milchsäurelaurylester ist keine ausführbare technische Lehre zur Herstellung von Laurylestern von Milchsäureoligomeren. So gelingt es bereits nicht, die Veresterung unter Verwendung von Toluol als Schleppmittel, welches mit dem bei der Reaktion freiwerdenden Wasser ein Azeotrop mit einem Siedepunkt von etwa 80 bis 90 °C bildet, unter Verwendung eines Wasserabscheiders auf die angegebenen 225 °C aufzuheizen. Dieses Dokument lehrt auch nicht, dass sich nicht sulfatierte Hydroxycarbonsäureester, geschweige denn Ester von Hydroxycarbonsäureoligomeren, als Verdickungsmittel eignen.

Die US 3,098,795 beschreibt kosmetische Zusammensetzungen für topische Anwendungen, die Milchsäureester von Fettalkoholen enthalten. Dieses Dokument erwähnt weder konkret Ester von Milchsäureoligomeren noch einen Einsatz als Verdickungsmittel.

Der vorliegenden Erfindung liegt die Aufgabe zugrunde, neue Verbindungen bereitzustellen, die sich vorteilhaft als Verdickungsmittel für diverse Anwendungen eignen. Sie sollen speziell geeignet sein, ein komplexes Anforderungsspektrum abzudecken, wie es eingangs beschrieben ist. Insbesondere soll es möglich sein, Verdickungsmittel für tensidhaltige Formulierungen bereitzustellen, die über gute Rheologie-modifizierende Eigenschaften verfügen, die zumindest vergleichbar sind mit aus dem Stand der Technik bekannten Verdickern auf Basis von petrochemischen Komponenten und/oder auf Basis von Alkylenoxiden.

Überraschenderweise wurde nun gefunden, dass diese Aufgabe durch Ester von Oligohydroxycarbonsäuren gelöst wird.

### ZUSAMMENFASSUNG DER ERFINDUNG

Ein erster Gegenstand der Erfindung sind Verbindungen der allgemeinen Formel (I) 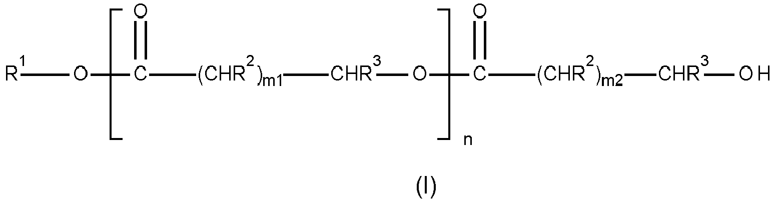 worin
- R¹: für Wasserstoff oder einen linearen oder verzweigten aliphatischen Kohlenwasserstoffrest mit 1 bis 30 Kohlenstoffatomen und 0, 1, 2 oder 3 Doppelbindungen steht,
- die Reste R²: unabhängig voneinander ausgewählt sind unter Wasserstoff, Methyl, Ethyl, -OH, -COOR⁴, -CH₂-OH und -CH₂-COOR⁴, wobei die Reste R⁴ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
- die Reste R³: unabhängig voneinander ausgewählt sind unter Wasserstoff, Methyl, Ethyl, -OH, -COOR⁵, -CH₂-OH und -CH₂-COOR⁵, wobei die Reste R⁵ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
- n: im Mittel für einen Wert von wenigstens 0,1 steht,
- m1 und m2: unabhängig voneinander für 0 oder 1 stehen,
mit der Maßgabe, dass wenigstens einer der Reste R¹, R⁴ oder R⁵ für einen linearen oder verzweigten aliphatischen Kohlenwasserstoffrest mit 1 bis 30 Kohlenstoffatomen und 0, 1, 2 oder 3 Doppelbindungen steht.

Eine bevorzugte Ausführungsform sind Ester von Oligolactaten. Danach sind die Verbindungen der allgemeinen Formel (I) ausgewählt unter Verbindungen der Formel (I.1) 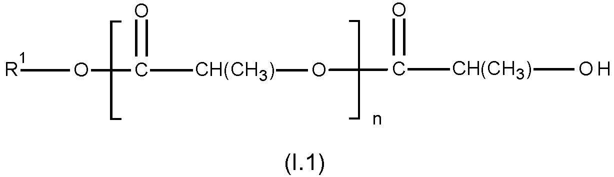 worin
- R¹: für Wasserstoff oder einen linearen oder verzweigten Kohlenwasserstoffrest mit 1 bis 30 Kohlenstoffatomen und 0, 1, 2 oder 3 Doppelbindungen steht, und
- n: im Mittel für einen Wert von wenigstens 0,1 steht.

Ein weiterer Gegenstand der Erfindung ist ein Verfahren zur Herstellung von Verbindungen der allgemeinen Formel (I). Gegenstand der Erfindung sind auch die nach diesem Verfahren erhältlichen Verbindungen der allgemeinen Formel (I).

Ein weiterer Gegenstand der Erfindung ist eine kosmetische oder pharmazeutische Zusammensetzung, die wenigstens eine Verbindung der allgemeinen Formel (I), wie zuvor und im Folgenden definiert, enthält.

Ein weiterer Gegenstand der Erfindung ist die Verwendung von Verbindungen der allgemeinen Formel (I), wie zuvor und im Folgenden definiert, als Verdicker für wässrige tensidhaltige Zusammensetzungen.

Ein weiterer Gegenstand der Erfindung ist die Verwendung von Verbindungen der allgemeinen Formel (I), wie zuvor und im Folgenden definiert, als Komponente zur Formulierung von
- kosmetischen Mitteln,
- pharmazeutischen Mitteln,
- Wasch- und Reinigungsmitteln,
- Pflanzenschutzmitteln,
- Netzmitteln,
- Lacken, Beschichtungsmitteln, Klebstoffen, Lederbehandlungs- oder Textilbehandlungsmitteln, etc.

### BESCHREIBUNG DER ERFINDUNG

Die Verbindungen der allgemeinen Formel (I) können in Form von Mischungen oder als Reinverbindungen vorliegen. Für die erfindungsgemäßen Verwendungen eignen sich in der Regel Mischungen von Verbindungen der allgemeinen Formel (I), wie sie z. B. durch das im Folgenden beschriebene Herstellungsverfahren erhältlich sind. Die einzelnen Komponenten dieser Mischungen können sich beispielsweise hinsichtlich des Oligomerisierungsgrads n unterscheiden. Werden zur Herstellung der Verbindungen der allgemeinen Formel (I) Hydroxycarbonsäuren eingesetzt, die mehr als eine Carboxylgruppe und/oder mehr als eine alkoholische OH-Gruppe aufweisen, so kann es sich bei den einzelnen Komponenten dieser Mischungen auch um Strukturisomere aus der Veresterungsreaktion zu ihrer Herstellung handeln. Selbstverständlich ist es auch möglich, die nach dem erfindungsgemäßen Verfahren erhältlichen Reaktionsgemische nach üblichen Trennverfahren, z. B. destillativ oder chromatographisch, aufzutrennen.

Der mittlere Oligomerisierungsgrad ergibt sich für die erfindungsgemäßen Verbindungen der allgemeinen Formel (I) und der allgemeinen Formel (I.1) durch Addition von 1 zum Wert der Variablen n.

Geeignete lineare oder verzweigte aliphatische Kohlenwasserstoffreste mit 1 bis 30 Kohlenstoffatomen und 0, 1, 2 oder 3 Doppelbindungen sind die entsprechenden C₁-C₃₀-Alkylreste, C₁-C₃₀-Alkenylreste, C₁-C₃₀-Alkadienylreste und C₁-C₃₀-Alkatrienyl-reste.

Bevorzugt steht wenigstens einer der Reste R¹, R⁴ oder R⁵ für einen linearen oder verzweigten aliphatischen Kohlenwasserstoffrest mit 1 bis 30 Kohlenstoffatomen und 0, 1, 2 oder 3 Doppelbindungen. Insbesondere sind R¹, R⁴ und R⁵ unabhängig voneinander ausgewählt unter Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2 Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, Isotridecyl, Isostearyl, Oleyl, Linoleyl, Linolenyl, etc.

In einer bevorzugten Ausführung steht wenigstens einer der Reste R¹, R⁴ oder R⁵ für einen linearen oder verzweigten aliphatischen Kohlenwasserstoffrest mit 6 bis 30 Kohlenstoffatomen und 0, 1, 2 oder 3 Doppelbindungen. Besonders bevorzugt sind R¹, R⁴ und R⁵ unabhängig voneinander ausgewählt unter n-Hexyl, 2-Ethylhexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, Isotridecyl, Isostearyl, Oleyl, Linoleyl, Linolenyl, und Kombinationen davon.

Die Reste R¹, R⁴ und R⁵ können sich von reinen Alkoholen oder von Alkoholgemischen ableiten. Bevorzugt handelt es sich um großtechnische verfügbare Alkohole oder Alkoholgemische. In einer bevorzugten Ausführungsform sind R¹, R⁴ und R⁵ dann unabhängig voneinander ausgewählt unter überwiegend linearen Alkyl-, Alkenyl-, Alkadienyl- und Alkatrienylresten, wie sie in natürlichen oder synthetischen Fettsäuren und den entsprechenden Fettalkoholen vorkommen.

In einer weiteren bevorzugten Ausführungsform sind R¹, R⁴ und R⁵ unabhängig voneinander abgeleitet von Fettalkoholen, die auf technischen Alkoholmischungen basieren. Dazu zählen z. B. die bei der Hydrierung von technischen Methylestern auf Basis von Fetten und Ölen anfallenden Alkoholgemische. Dazu zählen weiterhin die bei der Hydrierung von Aldehyden aus der Oxosynthese anfallenden Alkoholgemische (Oxoalkohole) oder die bei der Dimerisierung von ungesättigten Fettalkoholen anfallenden Alkoholgemische.

Bevorzugt leitet sich wenigstens einer der Reste R¹, R⁴ und R⁵ von linearen gesättigten Alkoholen mit 8 bis 18 Kohlenstoffatomen ab.

Besonders bevorzugt leitet sich wenigstens einer der Reste R¹, R⁴ und R⁵ von einem Gemisch linearer gesättigter C₁₂-/C₁₄-Alkohole ab.

Des Weiteren bevorzugt leitet sich wenigstens einer der Reste R¹, R⁴ und R⁵ von einem C₁₆-/C₁₈-Fettalkoholgemisch ab. Gemische aus Cetyl (Hexadecyl) und Stearyl (Octadecyl) werden auch als Cetearyl bezeichnet.

Bevorzugt haben in den Verbindungen (I) die Variablen m1 und m2 die gleiche Bedeutung.

Bei den Verbindungen der allgemeinen Formel (I) handelt es sich um Ester von Oligohydroxycarbonsäuren. Diese können sich von üblichen Hydroxycarbonsäuren ableiten, wie Milchsäure, Glycolsäure, Äpfelsäure, Weinsäure, Tartronsäure und Mischungen davon. Bevorzugt sind die Verbindungen (I) abgeleitet von Milchsäure, Glycolsäure, Äpfelsäure, Weinsäure oder Mischungen davon. Besonders bevorzugt sind die Verbindungen (I) abgeleitet von Milchsäure.

In den Verbindungen der allgemeinen Formel (I) steht n vorzugsweise für einen Wert von 0,1 bis 100, besonders bevorzugt von 0,15 bis 50, insbesondere von 0,2 bis 20.

In den Verbindungen der allgemeinen Formel (I.1) steht n vorzugsweise für einen Wert von 0,1 bis 100, besonders bevorzugt von 0,15 bis 50, insbesondere von 0,2 bis 20.

Ein weiterer Gegenstand der Erfindung ist ein Verfahren zur Herstellung von Verbindungen der allgemeinen Formel (I), bei dem man wenigstens eine Hydroxycarbonsäure der allgemeinen Formel (I.A) 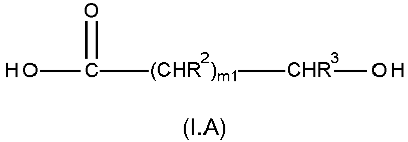 worin
- die Reste R²: unabhängig voneinander ausgewählt sind unter Wasserstoff, Methyl, Ethyl, -OH, -COOR⁴, -CH₂-OH und -CH₂-COOR⁴, wobei die Reste R⁴ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
- die Reste R³: unabhängig voneinander ausgewählt sind unter Wasserstoff, Methyl, Ethyl, -OH, -COOR⁵, -CH₂-OH und -CH₂-COOR⁵, wobei die Reste R⁵ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
- m1: für 0 oder 1 steht,

in einer Veresterungsreaktion umsetzt, wobei die Veresterung in Gegenwart wenigstens eines Alkohols R¹-OH erfolgt, wobei
- R¹: für Wasserstoff oder einen linearen oder verzweigten aliphatischen Kohlenwasserstoffrest mit 1 bis 30 Kohlenstoffatomen und 0, 1, 2 oder 3 Doppelbindungen steht,
oder das Produkt der Veresterung der Hydroxycarbonsäure(n) (I.A) anschließend mit wenigstens einem Alkohol R¹-OH umgesetzt wird.

Bezüglich geeigneter und bevorzugter Definitionen von R¹, R², R³, R⁴, R⁵ und m1 wird auf die vorherigen Ausführungen zu diesen Resten und Variablen in vollem Umfang Bezug genommen.

Die Veresterungsreaktion kann mehrstufig erfolgen, wobei zuerst wenigstens eine Hydroxycarbonsäure (I.A) einer Veresterung unter Oligomerisierung unterzogen wird und anschließend das erhaltene Reaktionsgemisch, gegebenenfalls nach einer Auftrennung und/oder Reinigung, in einer weiteren Veresterung mit wenigstens einem Alkohol R¹-OH umgesetzt wird.

Vorzugsweise erfolgt die Veresterungsreaktion zur Herstellung von Verbindungen der allgemeinen Formel (I) im Sinne einer Eintopfreaktion, bei der man wenigstens eine Hydroxycarbonsäure der allgemeinen Formel (I.A) in Gegenwart wenigstens eines Alkohols R¹-OH einer Veresterung unterzieht.

Die Veresterungsreaktion kann nach allgemein bekannten Verfahren erfolgen, wobei die Entfernung des gebildeten Reaktionswassers, z. B. durch wasserentziehende Mittel, extraktiv oder destillativ erfolgen kann.

Vorzugsweise wird das gebildete Reaktionswasser destillativ entfernt. In einer speziellen Ausführungsform kann das gebildete Reaktionswasser azeotrop entfernt werden. Die Umsetzung erfolgt dabei in Gegenwart eines Lösungsmittels, das mit Wasser ein azeotropes Gemisch bildet. Geeignete Lösungs- und Schleppmittel sind aliphatische und aromatische Kohlenwasserstoffe, z. B. Alkane, wie n-Hexan und n-Heptan, Cycloalkane, wie Cyclohexan und Methylcyclohexan, Aromaten, wie Benzol, Toluol und Xylolisomere und sogenannte Spezialbenzine, welche Siedepunkte zwischen 70 und 140 °C aufweisen. Besonders bevorzugte Schleppmittel sind Cyclohexan, Methylcyclohexan und Toluol. Geeignete Apparaturen zur azeotropen Destillation unter Abscheidung des Reaktionswassers und Rückführung des Lösungsmittels in das Reaktionsgefäß sind dem Fachmann bekannt. Das eingesetzte Lösungsmittel kann nach der Veresterung durch übliche Methoden, wie z. B. durch Destillation, gegebenenfalls unter vermindertem Druck, aus dem Reaktionsgemisch entfernt werden.

Wird zur Veresterung ein Alkohol R¹-OH mit ausreichend hohem Siedepunkt eingesetzt, z. B. ein gesättigter oder ein- oder mehrfach ungesättigter Fettalkohol mit wenigstens 6 Kohlenstoffatomen, so kann bei der destillativen Entfernung des Reaktionswassers auf den Einsatz eines Schleppmittels verzichtet werden.

Die Veresterungstemperatur liegt im Allgemeinen in einem Bereich von etwa 50 bis 250 °C, besonders bevorzugt von 70 bis 200 °C.

Die Veresterung erfolgt vorzugsweise bei Normaldruck oder vermindertem Druck. Zur destillativen Abtrennung des Reaktionswassers ist es vorteilhaft, die Veresterung bei vermindertem Druck durchzuführen. Bevorzugt liegt der Druck bei der Veresterung in einem Bereich von 1 mbar bis 1,1 bar, insbesondere 5 mbar bis 1 bar, speziell 10 mbar bis 900 mbar. Dies gilt sowohl für die zuvor beschriebene einstufige als auch die zweistufige Variante der Veresterungsreaktion.

Die Veresterung kann autokatalytisch oder in Gegenwart eines Katalysators erfolgen. Geeignete Katalysatoren sind starke Säuren, wie z. B. Schwefelsäure, wasserfreier Chlorwasserstoff, Sulfonsäuren, z. B. Toluolsulfonsäure und Methansulfonsäure, und saure Ionenaustauscher. Im erfindungsgemäßen Verfahren wird vorzugsweise Schwefelsäure und p-Toluolsulfonsäure als Katalysator eingesetzt. Die Menge des Veresterungskatalysators liegt dabei im Allgemeinen in einem Bereich von etwa 0,1 bis 5 Gew.-%, bezogen auf die Gesamtmenge an zu veresternden Komponenten.

Bevorzugt erfolgt die Veresterungsreaktion ohne Zusatz eines externen Lösungsmittels. Dies gilt sowohl für die zuvor beschriebene einstufige als auch die zweistufige Variante der Veresterungsreaktion. Es ist jedoch alternativ möglich, die erfindungsgemäße Umsetzung in Gegenwart eines unter den Reaktionsbedingungen inerten, organischen Lösemittels oder eines Lösungsmittelgemischs durchzuführen. Bevorzugt sind aprotische Lösemittel. Die eingesetzten Lösungsmittel weisen vorzugsweise einen Siedepunkt von wenigstens 120 °C, insbesondere von wenigstens 140 °C auf. Dazu zählen z. B. Alkylenglycoldialkylether wie Ethylenglykoldimethylether, Ethylenglykoldiethylether, etc. Ebenfalls geeignet sind z. B. Cyclohexanon (Sdp.: 155 °C), N-Methylpyrrolidon (Sdp.: 204 °C), Sulfolan (Sdp.: 285 °C), Nitrobenzol (Sdp.: 210 °C), Xylol (Sdp.: 140 °C).

Die Einstellung des gewünschten mittleren Oligomerisierungsgrads (p = 1 + n) gelingt in fachüblicher Weise.

So kann bei der zuvor beschriebenen zweistufigen Variante der Veresterungsreaktion zunächst eine oligomere Hydroxycarbonsäure mit dem gewünschten mittleren Oligomerisierungsgrad (p = 1 + n) hergestellt werden. Dazu wird das Reaktionsgemisch unter wasserentziehenden Bedingungen für einen Zeitraum bei der Reaktionstemperatur belassen, der ausreicht, um den angestrebten mittleren Oligomerisierungsgrad zu erreichen. Vorzugsweise beträgt die Reaktionsdauer etwa 0,5 bis etwa 24 Stunden, insbesondere etwa 1 bis etwa 20 Stunden. Anschließend wird die so erhaltene oligomere Hydroxycarbonsäure mit wenigstens einem Alkohol R¹-OH zum Endprodukt umgesetzt. Das molare Verhältnis von Alkoholkomponente R¹-OH zu oligomerer Hydroxycarbonsäure beträgt bei dieser Variante etwa 1 : 1.

Bei der zuvor beschriebenen einstufigen Variante der Veresterungsreaktion wird wenigstens eine Hydroxycarbonsäure der allgemeinen Formel (I.A) in Gegenwart wenigstens eines Alkohols R¹-OH einer Veresterung unterzogen. Nach dieser Variante gelingt die Einstellung des gewünschten mittleren Oligomerisierungsgrads n beispielsweise über das molare Verhältnis von Alkoholkomponente R¹-OH zu Hydroxycarbonsäure (I.A). Dieses beträgt bei dieser Variante bevorzugt 1 : 1,01 bis 1 : 200, besonders bevorzugt 1 : 1,1 bis 1 : 100, insbesondere 1 : 1,15 bis 1 : 50, speziell 1 : 1,2 bis 1 : 20. Auch nach dieser Variante wird das Reaktionsgemisch unter wasserentziehenden Bedingungen für einen Zeitraum bei der Reaktionstemperatur belassen, der ausreicht, um Verbindungen der allgemeinen Formel (I) mit dem angestrebten mittleren Oligomerisierungsgrad zu erreichen. Vorzugsweise beträgt die Reaktionsdauer etwa 0,5 bis etwa 24 Stunden, insbesondere etwa 1 bis etwa 20 Stunden.

Die Verbindungen der allgemeinen Formel (I) und der allgemeinen Formel (I.1) eignen sich in vorteilhafter Weise zur Modifizierung der rheologischen Eigenschaften wässriger Zusammensetzungen. Es kann sich ganz allgemein beispielsweise um kosmetische Zusammensetzungen, pharmazeutische Zusammensetzungen, Hygieneprodukte, Anstrichmittel, Zusammensetzungen für die Papierindustrie sowie die Textilindustrie handeln.

Die Verbindungen der allgemeinen Formel (I) und der allgemeinen Formel (I.1) eignen sich bevorzugt zur Verdickung der Konsistenz von tensidhaltigen wässrigen Zusammensetzungen in einem weiten Bereich. Sie fungieren dabei speziell als sogenannte "mizellare Verdicker", d. h. grenzflächenaktive Verbindungen, die zur Erhöhung der Viskosität von tensidhaltigen Formulierungen eingesetzt werden. Je nach Grundkonsistenz der flüssigen Zusammensetzungen können in Abhängigkeit von der Einsatzmenge des Copolymers in der Regel Fließeigenschaften von dünnflüssig bis hin zu fest (im Sinne von "nicht mehr fließend") erzielt werden.

### Tensidhaltige Zusammensetzung

Die erfindungsgemäßen Verbindungen der allgemeinen Formel (I) bzw. der allgemeinen Formel (I.1) eignen sich besonders vorteilhaft zur Formulierung tensidhaltiger Zusammensetzungen. Insbesondere handelt es sich dabei um wässrige tensidhaltige Zusammensetzungen. Die Verbindungen (I) bzw. (I.1) zeichnen sich in derartigen Zusammensetzungen durch ihre gute Verdickungswirkung auch bei geringen Einsatzmengen aus.

Die erfindungsgemäßen tensidhaltigen Zusammensetzungen weisen vorzugsweise einen Gesamttensidgehalt von 0,1 bis 75 Gew.-%, besonders bevorzugt von 0,5 bis 60 Gew.-%, insbesondere von 1 bis 50 Gew.-%, bezogen auf das Gesamtgewicht der tensidhaltigen Zusammensetzung, auf. Speziell liegt der Gesamttensidgehalt in einem Bereich von 5 bis 20 Gew.-%.

Geeignete Tenside sind anionische Tenside, nichtionische Tenside, kationische Tenside, amphotere Tenside und Mischungen davon.

Typische Beispiele für anionische Tenside sind Seifen, Alkylsulfonate, Alkylbenzolsulfonate, Olefinsulfonate, Alkylethersulfonate, Glycerinethersulfonate, Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acylglutamate und Acylaspartate, sowie Acyllactylate, Acyltartrate, Alkyloligoglucosidsulfate, Alkylglucosecarboxylate, Proteinfettsäurekondensate und Alkyl-(ether)phosphate.

Geeignete Seifen sind z. B. Alkali-, Erdalkali- und Ammoniumsalze von Fettsäuren, wie Kaliumstearat.

Geeignete Olefinsulfonate werden z. B. durch Anlagerung von SO₃ an Olefine der Formel R³-CH=CH-R⁴ und nachfolgende Hydrolyse und Neutralisation erhalten, wobei R³ und R⁴ unabhängig voneinander für H oder Alkylreste mit 1 bis 20 Kohlenstoffatomen stehen, mit der Maßgabe, dass R³ und R⁴ zusammen mindestens 6 und vorzugsweise 8 bis 20, speziell 10 bis 16, Kohlenstoffatome aufweisen. Hinsichtlich Herstellung und Verwendung sei auf den Übersichtsartikel "J. Am. Oil. Chem. Soc.", 55, 70 (1978) verwiesen. Die Olefinsulfonate können als Alkali-, Erdalkali-, Ammonium-, Alkylammonium-, Alkanolammonium- oder Glucammoniumsalze vorliegen. Vorzugsweise liegen die Olefinsulfonate als Natriumsalze vor. Das hydrolysierte alpha-Olefinsulfonierungsprodukt, d. h. die alpha-Olefinsulfonate, setzen sich aus ca. 60 Gew.-% Alkansulfonaten und ca. 40 Gew.-% Hydroxyalkansulfonaten zusammen; hiervon sind etwa 80 bis 85 Gew.-% Mono- und 15 bis 20 Gew.-% Disulfonate.

Bevorzugte Methylestersulfonate (MES) werden durch Sulfonierung der Fettsäuremethylester von pflanzlichen oder tierischen Fetten oder Ölen gewonnen. Bevorzugt sind Methylestersulfonate aus pflanzlichen Fetten und Ölen, z. B. aus Rapsöl, Sonnenblumenöl, Sojaöl, Palmöl, Kokosfett, etc.

Bevorzugte Alkylsulfate sind Sulfate von Fettalkoholen der allgemeinen Formel R⁶-O-SO₃Y, worin R⁶ für einen linearen oder verzweigten, gesättigten oder ungesättigten Kohlenwasserstoffrest mit 6 bis 22 Kohlenstoffatomen und Y für ein Alkalimetall, das einwertige Ladungsäquivalent eines Erdalkalimetalls, Ammonium-, Mono-, Di-, Tri- oder Tetraalkylammonium, Alkanolammonium oder Glucammonium steht. Geeignete Fettalkoholsulfate werden vorzugsweise durch Sulfatierung von nativen Fettalkoholen oder synthetischen Oxoalkoholen und nachfolgender Neutralisation erhalten werden. Typische Beispiele für Fettalkoholsulfate sind die Sulfatierungsprodukte des Capronalkohols, Caprylalkohols, 2-Ethylhexylalkohols, Caprinalkohols, Laurylalkohols, Isotridecylalkohols, Myristylalkohols, Cetylalkohols, Palmoleylalkohols, Stearylalkohols, Isostearylalkohols, Oleylalkohols, Elaidylalkohols, Petroselinylalkohols, Linolylalkohols, Linolenylalkohols, Behenylalkohols und Elaeostearylalkohols sowie die Salze und Mischungen davon. Bevorzugte Salze der Fettalkoholsulfate sind die Natrium- und Kaliumsalze, insbesondere die Natriumsalze. Bevorzugte Mischungen der Fettalkoholsulfate basieren auf technischen Alkoholmischungen, die z. B. bei der Hochdruckhydrierung von technischen Methylestern auf Basis von Fetten und Ölen oder bei der Hydrierung von Aldehyden aus der Oxosynthese oder bei der Dimerisierung von ungesättigten Fettalkoholen anfallen. Vorzugsweise werden zur Herstellung von Alkylsulfaten Fettalkohole und Fettalkoholgemische mit 12 bis 18 Kohlenstoffatomen und insbesondere 16 bis 18 Kohlenstoffatomen eingesetzt. Typische Beispiele hierfür sind technische Alkoholsulfate auf Basis von pflanzlichen Rohstoffen.

Bevorzugte Sarkosinate sind Natriumlauroylsarkosinat oder Natriumstearoylsarkosinat.

Bevorzugte Proteinfettsäurekondensate sind pflanzliche Produkte auf Weizenbasis.

Bevorzugte Alkylphosphate sind Mono- und Diphosphorsäurealkylester.

Geeignete Acylglutamate sind Verbindungen der Formel (I) 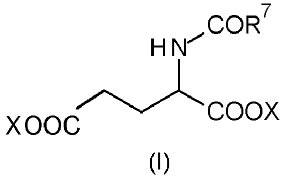 worin COR⁷ für einen linearen oder verzweigten Acylrest mit 6 bis 22 Kohlenstoffatomen und 0, 1, 2 oder 3 Doppelbindungen und X für Wasserstoff, ein Alkalimetall, das einwertige Ladungsäquivalent eines Erdalkalimetalls, Ammonium, Alkylammonium, Alkanolammonium oder Glucammonium steht. Die Herstellung von Acylglutamaten erfolgt beispielsweise durch Schotten-Baumann-Acylierung von Glutaminsäure mit Fettsäuren, Fettsäureestern oder Fettsäurehalogeniden. Acylglutamate sind kommerziell beispielsweise von der BASF SE, Clariant AG, Frankfurt/DE, oder der Ajinomoto Co. Inc., Tokio/JP, erhältlich. Eine Übersicht zu Herstellung und Eigenschaften der Acylglutamate findet sich von M. Takehara et al. in J. Am. Oil Chem. Soc. 49 (1972) 143. Typische als Komponente b) geeignete Acylglutamate leiten sich bevorzugt von Fettsäuren mit 6 bis 22 und besonders bevorzugt 12 bis 18 Kohlenstoffatomen ab. Es werden insbesondere die Mono- oder Dialkalimetallsalze des Acylglutamats eingesetzt. Dazu zählen z. B. (Handelsnamen der Fa. Ajinomoto, USA in Klammern): Natriumcocoylglutamat (Amisoft CS-11), Dinatriumcocoylglutamat (Amisoft ECS-22SB), Triethanolammoniumcocoylglutamat (Amisoft CT-12), Triethanolammoniumlauroylglutamat (Amisoft LT-12), Natriummyristoylglutamat (Amisoft MS-11), Natriumstearoylglutamat (Amisoft HS-11 P) und Mischungen davon.

Zu den nichtionischen Tensiden gehören beispielsweise:
- Fettalkoholpolyoxyalkylenester, beispielsweise Laurylalkoholpolyoxyethylenacetat,
- Alkylpolyoxyalkylenether, die sich von niedermolekularen C₁-C₆-Alkoholen oder von C₇-C₃₀-Fettalkoholen ableiten. Dabei kann die Etherkomponente aus Ethylenoxideinheiten, Propylenoxideinheiten, 1,2-Butylenoxideinheiten, 1,4-Butylenoxideinheiten und statistischen Copolymeren und Blockcopolymeren davon abgeleitet sein. Dazu zählen speziell Fettalkoholalkoxylate und Oxoalkoholalkoxylate, insbesondere vom Typ RO-(R⁸O)ᵣ(R⁹O)ₛR¹⁰ mit R⁸ und R⁹ unabhängig voneinander = C₂H₄, C₃H₆, C₄H₈ und R¹⁰ = H, oder C₁-C₁₂-Alkyl, R = C₃-C₃₀-Alkyl oder C₆-C₃₀-Alkenyl, r und s unabhängig voneinander 0 bis 50, wobei nicht beide für 0 stehen können, wie iso-Tridecylalkohol- und Oleylalkoholpolyoxyethylenether,
- Alkylarylalkohol-polyoxyethylenether, z. B. Octylphenol-polyoxyethylenether,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate. Beispielsweise können Cocodimethylsulfopropylbetain, Laurylbetain, Cocamidopropylbetain, Natriumcocamphopropionat oder Tetradecyldimethylaminoxid eingesetzt werden.

Zu den kationischen Tensiden gehören beispielsweise quaternierte Ammoniumverbindungen, insbesondere Alkyltrimethylammonium- und Dialkyldimethylammonium-halogenide und -alkylsulfate sowie Pyridin- und Imidazolin-Derivate, insbesondere Alkylpyridinium-Halogenide. Beispielsweise können Behenyl oder Cetyltrimethylammoniumchlorid eingesetzt werden. Geeignet sind weiterhin sogenannte Esterquats, die auf quartären Triethanol-Methyl-Ammonium- oder quartären Diethanol-Dimethyl-Ammonium-Verbindungen mit langen Kohlenwasserstoffketten in Form von Fettsäureestern basieren. Dazu zählt beispielsweise Bis(acyloxyethyl)hydroxyethylammonium Methosulfat. Geeignet ist weiterhin Dehyquart L 80 (INCI: Dicocoylethyl Hydroxyethylmonium Methosulfate (and) Propylene Glycol).

### Kosmetische und pharmazeutische Mittel

Die Verbindungen der allgemeinen Formel (I) und der allgemeinen Formel (I.1) eignen sich vorzugsweise zur Formulierung von kosmetischen und pharmazeutischen Produkten, speziell von wässrigen kosmetischen und pharmazeutischen Produkten.

Ein weiterer Gegenstand der Erfindung ist eine kosmetische oder pharmazeutische Zusammensetzung, enthaltend
a) wenigstens eine Verbindung der allgemeinen Formel (I), wie zuvor definiert,
b) wenigstens einen kosmetisch oder pharmazeutisch akzeptablen Wirkstoff, und
c) gegebenenfalls wenigstens einen von den Komponenten a) und b) verschiedenen kosmetisch oder pharmazeutisch akzeptablen Hilfsstoff.

Bevorzugt wird als Komponente a) wenigstens eine Verbindung der allgemeinen Formel (I.1) eingesetzt.

Bevorzugt umfasst die Komponente c) wenigstens einen kosmetisch oder pharmazeutisch akzeptablen Träger.

Vorzugsweise ist die Träger-Komponente c) ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Geeignete hydrophile Komponenten c) sind die zuvor genannten organischen Lösungsmittel, Öle und Fette.

Speziell geeignete kosmetisch verträgliche Öl- bzw. Fettkomponenten c) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Bei den erfindungsgemäßen kosmetischen Mitteln kann es sich um hautkosmetische, haarkosmetische, dermatologische, hygienische oder pharmazeutische Mittel handeln. Aufgrund ihrer verdickenden Eigenschaften eignen sich die zuvor beschriebenen Verbindungen der Formel (I) und der Formel (I.1) insbesondere als Zusatzstoffe für Haar- und Hautkosmetika.

Vorzugsweise liegen die erfindungsgemäßen Mittel in Form eines Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetischen Mittel können zusätzlich kosmetisch und/oder dermatologisch aktive Wirk- und Effektstoffe sowie Hilfsstoffe enthalten. Vorzugsweise enthalten die erfindungsgemäßen kosmetischen Mittel wenigstens eine Verbindung der Formel (I) oder der Formel (I.1), wie vorstehend definiert, wenigstens einen wie vorstehend definierten Träger C) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

Zusätzlich zu den Verbindungen der Formel (I) und der Formel (I.1) können die kosmetischen Mittel wenigstens ein konventionelles Verdickungsmittel enthalten. Dazu zählen z. B. Polysaccharide und organische Schichtmineralien wie Xanthan Gum^{®} (Kelzan^{®} der Fa. Kelco), Rhodopol^{®} 23 (Rhone Poulenc) oder Veegum^{®} (Firma R. T. Vanderbilt) oder Attaclay^{®} (Firma Engelhardt). Geeignete Verdickungsmittel sind auch organische natürliche Verdickungsmittel (Agar-Agar, Carrageen, Tragant, Gummi arabicum, Alginate, Pektine, Polyosen, Guar-Mehl, Johannisbrotbaumkernmehl, Stärke, Dextrine, Gelatine, Casein) und anorganische Verdickungsmittel (Polykieselsäuren, Tonmineralien wie Montmorillonite, Zeolithe, Kieselsäuren). Weitere Verdickungsmittel sind Polysaccharidgummen, beispielsweise Gummi arabicum, Agar, Alginate, Carrageene und ihre Salze, Guar, Guaran, Traganth, Gellan, Ramsan, Dextran oder Xanthan und ihre Derivate, z. B. propoxyliertes Guar, sowie ihre Mischungen. Andere Polysaccharidverdicker sind beispielsweise Stärken verschiedensten Ursprungs und Stärkederivate, z. B. Hydroxyethylstärke, Stärkephosphatester oder Stärkeacetate, oder Carboxymethylcellulose bzw. ihr Natriumsalz, Methyl-, Ethyl-, Hydroxyethyl-, Hydroxypropyl-, Hydroxypropyl-methyl- oder Hydroxyethyl-methyl-cellulose oder Celluloseacetat. Als Verdickungsmittel können weiterhin Schichtsilikate eingesetzt werden. Hierzu zählen beispielsweise die unter dem Handelsnamen Laponite ® erhältlichen Magnesium- oder Natrium-Magnesium-Schichtsilikate der Firma Solvay Alkali sowie die Magnesiumsilikate der Firma Süd-Chemie.

Geeignete kosmetisch und/oder dermatologisch aktive Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-Bund/oder UV-A-Bereich absorbieren. Geeignete UV-Filter sind die zuvor genannten. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeere, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen Wirkstoff (wie auch gegebenenfalls als Hilfsstoff) wenigstens ein kosmetisch oder pharmazeutisch akzeptables Polymer enthalten. Dazu zählen ganz allgemein anionische, kationische, amphotere und neutrale Polymere.

Beispiele für anionische Polymere sind Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der (Meth)acrylsäure, C₄-C₃₀-Alkylvinylester, C₄-C₃₀-Alkylvinylether und Hyaluronsäure. Ein Beispiel für ein anionisches Polymer ist weiterhin das unter der Bezeichnung Luviset® Shape (INCI Name: Polyacrylate-22) erhältliche Methylmethacrylat/Methacrylsäure/Acrylsäure/Urethanacrylat-Copolymer. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und -R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviset Clear®, Luviquat Supreme ®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF SE), oder Polyvinylpyrrolidon und deren Copolymere, insbesondere mit Vinylestern, wie Vinylacetat, z. B. Luviskol® VA 37, VA 55, VA 64, VA 73 (BASF SE); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

In einer speziellen Ausführung enthalten die erfindungsgemäßen Zusammensetzungen wenigstens ein Polymer, das als Verdicker wirkt.

Geeignete polymere Verdicker sind beispielsweise gegebenenfalls modifizierte polymere Naturstoffe (Carboxymethylcellulose und andere Celluloseether, Hydroxyethyl- und -propylcellulose und dergleichen) sowie synthetische polymere Verdicker (Polyacryl- und Polymethacryl-Verbindungen, Vinylpolymere, Polycarbonsäuren, Polyether, Polyimine, Polyamide). Dazu zählen die zum Teil bereits zuvor genannten Polyacryl- und Polymethacryl-Verbindungen, beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung: Carbomer). Solche Polyacrylsäuren sind u. a. von der Fa. BF Goodrich unter dem Handelsnamen Carbopol ® erhältlich, z. B. Carbopol 940 (Molekulargewicht ca. 4000000), Carbopol 941 (Molekulargewicht ca. 1250000) oder Carbopol 934 (Molekulargewicht ca. 3000000). Weiterhin fallen darunter Acrylsäure-Copolymere, die beispielsweise von der Fa. Rohm & Haas unter den Handelsnamen Aculyn ® und Acusol ® erhältlich sind, z. B. die anionischen, nicht-assoziativen Polymere Aculyn 22, Aculyne 28, Aculyn 33 (vernetzt), Acusol 810, Acusol 823 und Acusol 830 (CAS 25852-37-3). Speziell geeignet sind auch assoziative Verdicker, z.B. auf Basis modifizierter Polyurethane (HEUR) oder hydrophob modifizierter Acryl- oder Methacrylsäure-Copolymere (HASE-Verdicker, High Alkali Swellable Emulsion).

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die Verbindungen der Formel (I) und der Formel (I.1) verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Verbindungen der Formel (I) und der Formel (I.1) zeigen vorteilhafte Wirkungen. Die Polymere können unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens eine Verbindungen der Formel (I) und der Formel (I.1) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Verbindungen der Formel (I) und der Formel (I.1) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Polymere auch mit herkömmlichen Polymeren, wie zuvor beschrieben, abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen, insbesondere als Wasser-in-Öl-(W/O)- oder Öl-in-Wasser-(O/W)-Emulsionen, vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem Verbindungen der Formel (I) und der Formel (I.1) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann eine Verbindung der allgemeinen Formel (I) oder (I.1) eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karitö-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Es können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens eine Verbindung der allgemeinen Formel (I) oder (I.1) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/- Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete Tenside sind die zuvor genannten.

Weiterhin können die Duschgel-/Shampoo-Formulierungen zusätzliche Verdicker, wie z. B. Kochsalz, PEG-55, Propylenglykol-Oleat, PEG-120-Methylglucosedioleat und andere enthalten. Geeignete kommerziell erhältliche weitere Verdicker sind z.B. Arlypon TT (INCI: PEG/PPG-120/10 Trimethylolpropane Trioleate (and) Laureth-2) und Arlypon F (INCI: Laureth-2). Des Weiteren können die Duschgel-/Shampoo-Formulierungen Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens eine Verbindung der allgemeinen Formel (I) oder (I.1) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 5 Gew.-%, vorzugsweise 0,1 bis 3 Gew.-%, wenigstens einer Verbindung der allgemeinen Formel (I) oder (I.1),
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0,05 bis 5 Gew.-% wenigstens eines kosmetisch aktiven Wirkstoffs, sowie
f) 0 bis 20 Gew.-%, vorzugsweise 0,1 bis 10 Gew.-%, wenigstens eines von a) bis e) und g) verschiedenen wasserlöslichen oder wasserdispergierbaren Polymers,
g) 0 bis 45 Gew.-%, vorzugsweise 0,05 bis 25 Gew.-%, weitere Bestandteile,
wobei sich die Komponenten a) bis g) zu 100 Gew.-% addieren.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Copolymerzusammensetzungen eignen sich insbesondere als Verdicker in Haarstyling-Zubereitungen, insbesondere Haarschäumen und Haargelen.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Die erfindungsgemäßen Verbindungen der Formel (I) und der Formel (I.1) eignen sich auch für Styling-Gele. Als zusätzliche Gelbildner können alle in der Kosmetik üblichen Gelbildner eingesetzt werden. Hierzu wird auf die zuvor genannten konventionellen Verdicker Bezug genommen.

Die erfindungsgemäßen Verbindungen der Formel (I) und der Formel (I.1) eignen sich auch für Shampooformulierungen, die zusätzlich übliche Tenside enthalten.

In den Shampooformulierungen können zur Erzielung bestimmter Effekte übliche Konditioniermittel in Kombination mit den Verbindungen der Formel (I) und der Formel (I.1) eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die erfindungsgemäß zu verwendenden Verbindungen der Formel (I) und der Formel (1.1) eignen sich ebenso für die Verwendung zur Modifizierung der rheologischen Eigenschaften in pharmazeutischen Zubereitungen jeder Art.

Ein weiterer Gegenstand der Erfindung ist die Verwendung einer Verbindung der Formel (I) oder der Formel (I.1), wie zuvor definiert, als Hilfsstoff in der Pharmazie.

Typische pharmazeutische Zusammensetzungen enthalten
A) wenigstens eine einer Verbindung der Formel (I) oder der Formel (I.1), wie zuvor definiert,
B) wenigstens einen pharmazeutisch akzeptablen Wirkstoff und
C) gegebenenfalls wenigstens einen weiteren, von A) und B) verschiedenen pharmazeutisch akzeptablen Hilfsstoff.

Pharmazeutisch akzeptable Hilfsstoffe C) sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe C) können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, zusätzliche Verdicker, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung von erfindungsgemäßen pharmazeutischen Mitteln können die Wirkstoffe mit einem geeigneten Exzipienten vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendenden Copolymere auch zum Einsatz in oralen Darreichungsformen wie Tabletten, Kapseln, Pulvern, Lösungen. Hier können sie den schwerlöslichen Arzneistoff mit einer erhöhten Bioverfügbarkeit zur Verfügung stellen. Bei der parenteralen Applikation können neben Solubilisaten auch Emulsionen, beispielsweise Fettemulsionen, eingesetzt werden.

Pharmazeutische Formulierungen der oben genannten Art können durch Verarbeiten der erfindungsgemäß zu verwendenden Verbindungen der Formel (I) und der Formel (I.1) mit pharmazeutischen Wirkstoffen nach herkömmlichen Methoden und unter Einsatz bekannter und neuer Wirkstoffe erhalten werden.

Der Gehalt an wenigstens einer Verbindung der allgemeinen Formel (I) oder der allgemeinen Formel (I.1) in den pharmazeutischen Mitteln liegt, abhängig vom Wirkstoff, im Bereich von 0,01 bis 50 Gew.-%, bevorzugt 0,1 bis 40 Gew.-%, besonders bevorzugt 1 bis 30 Gew.-%, bezogen auf das Gesamtgewicht des Mittels.

Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Neben der Anwendung in der Kosmetik und in der Pharmazie eignen sich die erfindungsgemäß zu verwendenden Verbindungen der Formel (I) und der Formel (I.1) auch im Lebensmittelbereich zur Modifizierung der rheologischen Eigenschaften. Gegenstand der Erfindung sind daher auch lebensmitteltechnische Zubereitungen, die mindestens eine der erfindungsgemäß zu verwendenden Verbindungen der Formel (I) und der Formel (I.1) enthalten. Zu den Lebensmittelzubereitungen sind im Rahmen der vorliegenden Erfindung auch Nahrungsergänzungsmittel wie z. B. Lebensmittelfarbstoffe enthaltende Zubereitungen und diätetische Lebensmittel zu verstehen. Darüber hinaus eigenen sich die genannten Verbindungen der Formel (I) und der Formel (I.1) auch zur Modifizierung der rheologischen Eigenschaften für Futtermittelzusätze für die Tierernährung.

Außerdem eignen sich die Verbindungen der Formel (I) und der Formel (I.1) zur Herstellung wässriger Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

Allgemein können die erfindungsgemäßen Verbindungen der allgemeinen Formel (I) in allen Bereichen eingesetzt werden, in denen eine verdickende Wirkung in Kombination mit grenzflächenaktiven Stoffen notwendig ist.

Weiterhin sind die Verbindungen der allgemeinen Formel (I) geeignet, die Löslichkeit anderer Komponenten, z. B. anderer oberflächenaktiver Komponenten, wie von anionischen Tensiden, zu verbessern. Sie tragen somit auch positiv zur Bildung klarer tensidhaltiger Lösungen bei.

Die Erfindung wird anhand der folgenden nicht einschränkenden Beispiele näher erläutert.

### Beispiele

### A) Herstellungsbeispiele

### Herstellbeispiel 1:

(C_{12/14}-Alkyl)oligolactat (Oligomerisierungsgrad p = 1,2)

1744,4 g (9 Mol) eines C_{12/14} Fettalkoholgemischs (Lorol ® C_{12/14} spez. der BASF SE) (MG = 193,82 g/mol) und 1081,0 g (10,8 Mol) Milchsäure 90 % (MG = 90,08 g/mol) wurden in einer Destillationsapparatur unter Rühren zuerst unter Normaldruck und bei nachlassender Wasserabscheidung unter Vakuum bis 40 mbar auf 130 °C bis 150 °C erhitzt und freiwerdendes Reaktionswasser abdestilliert bis eine Säurezahl von unter 5 erreicht wurde (Dauer ca. 8 Stunden). Das Produkt hatte nach gaschromatographischer Analyse die folgende Zusamensetzung (Angaben in Flächen-%):

| Anzahl Milchsäureoligomere | GC-Flächen-% |
|---|---|
| 0 | 7,30 |
| 1 | 67,61 |
| 2 | 17,45 |
| 3 | 3,85 |
| 4 | 0,92 |
| 5 | 0,20 |
| 6 | 0,00 |

Aus messtechnischen Gründen addieren sich die ermittelten GC-Flächen-%-Werte nicht ganz zu 100 %. Die verbleibende geringe Restfläche gehört zu keinem der Milchsäureoligomere.

### Herstellungsbeispiele 2 bis 4:

In gleicher Weise wie Beispiel 1 wurden Produkte mit den Oligomerisierungsgraden p = 2, 3 und 5 hergestellt: Diese Produkte hatten nach gaschromatographischer Analyse die folgenden Zusammensetzungen (Angaben in Flächen-%):

| | | | |
|---|---|---|---|
| Beispiel Nr. | 2 | 3 | 4 |
| Oligomerisierungsgrad p | 2 | 3 | 5 |
| Anzahl Milchsäureoligomere | | | |
| 0 | 1,48 | 0,52 | 0,53 |
| 1 | 41,11 | 21,97 | 12,99 |
| 2 | 26,91 | 21,33 | 12,65 |
| 3 | 14,31 | 16,22 | 12,58 |
| 4 | 7,33 | 11,80 | 10,69 |
| 5 | 3,60 | 8,30 | 9,18 |
| 6 | 1,71 | 5,58 | 7,29 |
| 7 | 0,79 | 3,70 | 5,77 |
| 8 | 0,33 | 2,36 | 4,33 |
| 9 | 0,07 | 1,38 | 2,91 |
| 10 | 0,00 | 0,71 | 1,67 |
| 11 | 0,00 | 0,30 | 0,82 |
| 12 | 0,00 | 0,08 | 0,30 |

### Vergleichsbeispiel:

In gleicher Weise wie Beispiel 1 wurde ein Vergleichsprodukt mit einem Oligomerisierungsgraden p = 1,08 hergestellt: Dieses Produkt hatte nach gaschromatographischer Analyse die folgenden Zusammensetzungen (Angaben in Flächen-%):

| Anzahl Milchsäureoligomere | GC-Flächen-% |
|---|---|
| 0 | 2,2 |
| 1 | 86,8 |
| 2 | 9 |
| 3 | 0,68 |

### B) Anwendungstechnische Beispiele

Die Verdickungsleistung der Substanzen der erfindungsgemäßen Herstellungsbeispiele und des Vergleichsbeispiels wurde in verschiedenen Formulierungen getestet. Bestimmt wurde die Viskosität mit einem Viskosimeter: Brookfield DII + pro bei einer Messtemperatur von 22 °C.

### Beispiel I)

Formulierung:
99 g (= ca. 12 % Aktivsubstanz) Plantapon ® SF
1 g (C_{12/14}-Alkyl) (mono/oligo)lactat

(Plantapon ® SF: INCI: Sodium Cocoamphoacetate and Glycerin and Lauryl Glucosid and Sodium Cocoyl Glutamate and Sodium Lauryl Glucose Carboxylat)

Plantapon® SF basiert auf zwei anionischen Tensiden (unter 5 % Sodium Lauryl Glucose Carboxylat und unter 5 % Sodium Cocoyl Glutamate), einem amphoteren Tensid (10 - 15 % Sodium Cocoamphoacetate) und einem nichtionischen Tensid (5 bis 15 % Lauryl Glucoside) sowie 5 bis 15 % Glycerin)

| Substanz aus Beispiel Nr. | Vergleichsbeispiel | 1 |
|---|---|---|
| Viskosität [mPas]: | 1700 | 2070 |
| | Spindel S 64 | Spindel S 64 |
| | 60 rpm | 60 rpm |

### Beispiel 11)

Formulierung:
37,7 g (= ca. 10 % Aktivsubstanz) Texapon ® NSO (Natriumlaurylethersulphat mit 2 Mol EO)
5,3 g (= ca. 2 % Aktivsubstanz) Dehyton ® PK 45 (Cocamidopropylbetain) 2 g NaCl
1 g (C_{12/14}-Alkyl) (mono/oligo)lactat
Wasser ad 100 g

| Beispiel Nr. | Vergleichsbeispiel | 1 | 2 | 3 | 4 |
|---|---|---|---|---|---|
| Oligomerisierungsgrad p | 1,08 | 1,2 | 2 | 3 | 5 |
| Viskosität [mPas]: | 11717 | 21445 | 25645 | 28844 | 22795 |
| | Spindel S64 | Spindel S64 | Spindel S64 | Spindel S64 | Spindel S64 |
| | 12 rpm | 12 rpm | 12 rpm | 12 rpm | 12 rpm |

### Beispiel III)

Formulierung:
99 g (= ca. 12 % Aktivsubstanz) Plantapon ® SF
1 g (C_{12/14}-Alkyl) (mono/oligo)lactat

Messtemperatur T = 23 °C

| Beispiel Nr. | 1 | 2 | 3 | 4 |
|---|---|---|---|---|
| Oligomerisierungsgrad p | 1,2 | 2 | 3 | 5 |
| Viskosität [mPas]: | 1218 | 1626 | 7092 | 798 |
| | Spindel TC 93 | Spindel TC 93 | Spindel TC 93 | Spindel TC 93 |
| | 50 rpm | 20 rpm | 6 rpm | 60 rpm |

## Patentansprüche

1. Verbindungen der allgemeinen Formel (I) 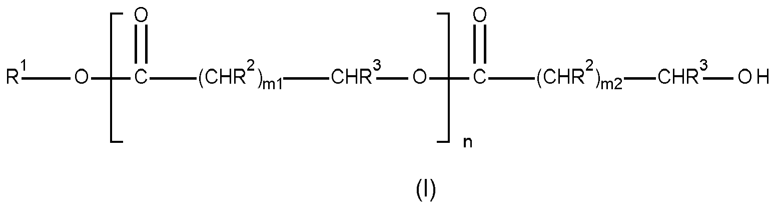 worin
R¹ für Wasserstoff oder einen linearen oder verzweigten aliphatischen Kohlenwasserstoffrest mit 1 bis 30 Kohlenstoffatomen und 0, 1, 2 oder 3 Doppelbindungen steht,
die Reste R² unabhängig voneinander ausgewählt sind unter Wasserstoff, Methyl, Ethyl, -OH, -COOR⁴, -CH₂-OH und -CH₂-COOR⁴, wobei die Reste R⁴ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
die Reste R³ unabhängig voneinander ausgewählt sind unter Wasserstoff, Methyl, Ethyl, -OH, -COOR⁵, -CH₂-OH und -CH₂-COOR⁵, wobei die Reste R⁵ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
n im Mittel für einen Wert von wenigstens 0,1 steht,
m1 und m2 unabhängig voneinander für 0 oder 1 stehen,
mit der Maßgabe, dass wenigstens einer der Reste R¹, R⁴ oder R⁵ für einen linearen oder verzweigten aliphatischen Kohlenwasserstoffrest mit 1 bis 30 Kohlenstoffatomen und 0, 1, 2 oder 3 Doppelbindungen steht.

2. Verbindungen (I) nach Anspruch 1, wobei m1 und m2 die gleiche Bedeutung haben.

3. Verbindungen (I) nach einem der vorhergehenden Ansprüche, die abgeleitet sind von Milchsäure, Glycolsäure, Äpfelsäure, Weinsäure oder Mischungen davon.

4. Verbindungen (I) nach einem der vorhergehenden Ansprüche, die ausgewählt sind unter Verbindungen der Formel (I.1) 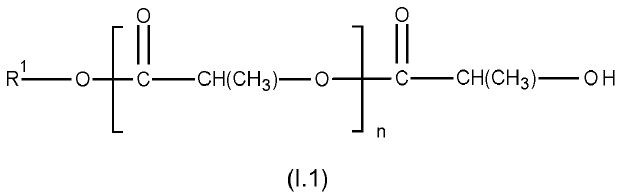 worin
R¹ für Wasserstoff oder einen linearen oder verzweigten Kohlenwasserstoffrest mit 1 bis 30 Kohlenstoffatomen und 0, 1, 2 oder 3 Doppelbindungen steht, und
n im Mittel für einen Wert von wenigstens 0,1 steht.

5. Verbindungen (I) nach einem der vorhergehenden Ansprüche, worin n für einen Wert von 0,1 bis 100, vorzugsweise von 0,15 bis 50, insbesondere von 0,2 bis 20, steht.

6. Verbindungen (I) nach einem der vorhergehenden Ansprüche, wobei wenigstens einer der Reste R¹, R⁴ und R⁵ für Methyl, Ethyl, n-Propyl, Isopropyl, n-Butyl, Isobutyl, sek.-Butyl, tert.-Butyl, n-Pentyl, Isoamyl, n-Hexyl, 2-Ethylhexyl, n-Heptyl, n-Octyl, n-Nonyl, n-Decyl, 2-Propylheptyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, Isotridecyl, Isostearyl, Oleyl, Linoleyl, Linolenyl oder eine Kombination aus wenigstens zwei dieser Reste steht.

7. Verbindungen (I) nach einem der vorhergehenden Ansprüche, wobei wenigstens einer der Reste R¹, R⁴ und R⁵ von linearen gesättigten Alkoholen mit 8 bis 18 Kohlenstoffatomen ableitet.

8. Verbindungen (I) nach einem der vorhergehenden Ansprüche, wobei wenigstens einer der Reste R¹, R⁴ und R⁵ von einem Gemisch linearer gesättigter C₁₂-/C₁₄-Alkohole ableitet.

9. Verfahren zur Herstellung von Verbindungen der allgemeinen Formel (I), bei dem man wenigstens eine Hydroxycarbonsäure der allgemeinen Formel (I.A) 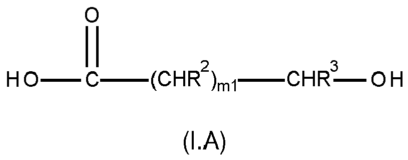 worin R², R³ und m1 wie in einem der Ansprüche 1 bis 8 definiert sind, in einer Veresterungsreaktion umsetzt, wobei die Veresterung in Gegenwart wenigstens eines Alkohols R¹-OH erfolgt, wobei R¹ wie in einem der Ansprüche 1 bis 8 definiert ist oder das Produkt der Veresterung der Hydroxycarbonsäure(n) (I.A) anschließend mit wenigstens einem Alkohol R¹-OH umgesetzt wird.

10. Verbindungen der allgemeinen Formel (I), wie in einem der Ansprüche 1 bis 8 definiert, erhältlich durch ein Verfahren, bei dem man wenigstens eine Hydroxycarbonsäure der allgemeinen Formel (I.A) 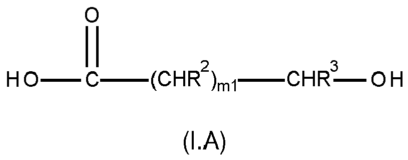 worin R², R³ und m1 wie in einem der Ansprüche 1 bis 8 definiert sind, in einer Veresterungsreaktion umsetzt, wobei die Veresterung in Gegenwart wenigstens eines Alkohols R¹-OH erfolgt, wobei R¹ wie in einem der Ansprüche 1 bis 8 definiert ist oder das Produkt der Veresterung der Hydroxycarbonsäure(n) (I.A) anschließend mit wenigstens einem Alkohol R¹-OH umgesetzt wird.

11. Kosmetische oder pharmazeutische Zusammensetzung, enthaltend
a) wenigstens eine Verbindung der allgemeinen Formel (I), wie in einem der Ansprüche 1 bis 8 oder 10 definiert,
b) wenigstens einen kosmetisch oder pharmazeutisch akzeptablen Wirkstoff, und
c) gegebenenfalls wenigstens einen von den Komponenten a) und b) verschiedenen kosmetisch oder pharmazeutisch akzeptablen Hilfsstoff.

12. Verwendung wenigstens einer Verbindung der allgemeinen Formel (I), wie in einem der Ansprüche 1 bis 8 oder 10 definiert, als Verdicker für wässrige tensidhaltige Zusammensetzungen.

13. Verwendung wenigstens einer Verbindung der allgemeinen Formel (I), wie in einem der Ansprüche 1 bis 8 oder 10 definiert, als Komponente zur Formulierung von
- kosmetischen Mitteln,
- pharmazeutischen Mitteln,
- Wasch- und Reinigungsmitteln,
- Pflanzenschutzmitteln,
- Netzmitteln,
- Lacken, Beschichtungsmitteln, Klebstoffen, Lederbehandlungs- oder Textilbehandlungsmitteln, etc.
